# EUROPEAN PATENT APPLICATION

(11) **EP 1 985 709 A1**
(43) Date of publication of application: **29.10.2008**
(21) Application number: 07450076.0
(22) Date of filing: 24.04.2007
(51) Int. Cl.: C12N 15/86

(54) **A method for expressing proteins**

(71) Applicant: Medizinische Universität Wien, 1090 Wien (AT)
(72) Inventor: Mandl, Christian, 1160 Vienna (AT); Heinz, Franz Xaver, 1170 Vienna (AT); Orlinger, Klaus, 1050 Vienna (AT); Kofler, Regina, 1230 Vienna (AT); Hönninger, Verena, 1190 Vienna (AT)
(74) Representative: Sonn & Partner Patentanwälte

(57) **Abstract**

Described is a method for expressing proteins comprising
- providing a bicistronic flavivirus expression construct comprising a 5'-terminal CAP structure, a 5'-non-coding sequence, a sequence encoding non-structural proteins, an internal ribosomal entry site (IRES) comprising an oligo(A) loop, a sequence encoding the protein to be expressed and a 3'-non-coding sequence in a suitable host cell and
- incubating this host cell with the expression construct to allow the protein to be expressed,

characterised in that the IRES carries a mutation in the oligo(A) loop, constructs with such mutated IRES and cells containing such constructs.

## Description

The present invention relates to a method for expressing proteins using bicistronic flavivirus expression constructs.

The virus family Flaviviridae comprises three genera flavivirus, pestivirus and hepacivirus. These genera have common genome structure, replication cycles and viral morphology; but differ in biological characteristics.

The genus flavivirus is very homogeneous; the structural and functional properties of the members of this genus are highly conserved. This genus includes several important arthropod-borne human pathogens including yellow fever virus, West Nile virus, Japanese encephalitis virus, dengue virus, and tick-borne encephalitis virus (TBEV). Mature flavivirus virions contain only three structural proteins. The capsid (or core) protein C associates with the viral genomic RNA to form the nucleocapsid, which is enveloped by a membrane into which the two surface proteins, E (envelope protein) and M (membrane protein), are embedded. Virions are assembled intracellularly in an immature form containing a larger glycoprotein precursor of the M protein, called prM. Cleavage of the precursor by a cellular protease activates viral entry functions and is required for infectivity.

The flavivirus genome is a single-stranded RNA molecule that functions as a messenger RNA for translation and is therefore infectious when introduced into the cell cytoplasm by transfection. This RNA is approximately 11 kilobases in length and has a 5' CAP structure, but lacks a polyA tail at the 3' end. All of the viral structural and non-structural proteins are encoded within a single open reading frame and a polyprotein is produced from this reading frame via CAP-dependent translation. The polyprotein is processed co- and posttranslationally in the host cell. The mature products (three structural proteins (C, prM-M and E) and seven non-structural proteins) are cleaved from this polyprotein via viral and cellular proteases. Additionally, the virus genome contains 5' and 3' non-coding regions regulating the replication and translation of the viral genome. After assembly of the viral replication complex the viral genome is replicated to more than 10,000 copies per cell. These mRNA molecules are then translated into viral proteins or serve as matrix for further replication or are packed into viral particles.

Efficient gene expression and a broad host range (including relatively low cytopathogenicity in various hosts) make flaviviruses an interesting tool for development of expression systems for heterologous genes. For example, efficient expression systems have been described for Kunjin virus, West Nile virus, Yellow fever virus and tick-borne encephalitis virus.

Despite the fact that the flavivirus genome contains only a single CAP-dependent long open reading frame, several studies have demonstrated that it is possible, by introducing a heterologous viral internal ribosome entry site (IRES) into the 3'-noncoding region (3'-NCR), to achieve expression from a separate, CAP-independent cistron, thus making it feasible to use modified flaviviruses as expression systems for foreign genes (Gehrke et al., J.Gen.Virol. 86 (2005), 1045-1053; WO 02/089840 A1). This means that in addition to the natural, CAP-translated reading frame of the flavivirus genome a further (IRES controlled) reading frame is present which can be used for heterologous proteins but also for viral proteins. The flaviviral genome obviously has a certain flexibility which preserves functionality of the system at least to a certain extent. In addition, it was demonstrated that it is possible to engineer viable flaviviruses in which the envelope glycoproteins prM and E are expressed from a separate translation unit, driven by a foreign IRES element, instead of as part of the natural CAP-dependent polyprotein (Orlinger et al., J. Virology 80 (2006), 12197-12208). The bicistronic flavivirus clone was made by replacing a variable region in the 3'-NCR of TBE virus with an IRES from encephalomyocarditis virus (EMCV), removing the portion of the genome encoding prM and E from its natural position, and inserting it into the 3'-NCR behind the IRES. This construct, called TBEV-bc (for bicistronic TBE virus), was able to replicate, make functional infectious virus particles, and to be propagated in cell culture and in animals. However, RNA replication, translation and (in the case of infectious vectors) possibly also packaging are severely hampered in such bicistronic systems. For example, TBEV-bc was significantly impaired in comparison to wild-type TBE virus, and in this genomic context, the prM and E proteins were expressed rather inefficiently from the IRES cistron.

Providing the IRES element creates functional disturbances in flaviviral vectors which negatively interfere with replication, expression levels and other functional parameters (Orlinger et al., J. Virology 80 (2006), 12197-12208). This has also been found in other, non-flaviviral bicistronic expression systems. Although IRES elements per se enable a significantly enhanced translation, this leads in the bicistronic context only to a low expression rate. This has also been confirmed for IRES controlled cistrons in flaviviral bicistronic systems. Orlinger et al. have shown that the expression rate of the CAP-translated cistron is reduced by the factor 2 and the expression rate of the IRES-translated cistron by the factor 100 (each in comparison to the monocistronic expression rate). It was also observed that the IRES element led to a decrease in RNA replication (directly correlating with the length of the IRES-controlled cistron. The problem of reduced expression efficiency is a major restriction in the applicability and practical utility of flaviviral vectors.

The present invention aims at overcoming the problems associated with bicistronic expression systems in flaviviral expression constructs. It is an object of the present invention to provide improved bicistronic flaviviral expression constructs. It is a further object to provide industrially usable bicistronic flavivirus expression systems which allow efficient protein expression especially for expression of heterologous recombinant proteins or for production of improved flavivirus vaccines.

Therefore, the present invention provides a method for expressing proteins comprising
- providing a bicistronic flavivirus expression construct comprising a 5'-terminal CAP structure, a 5'-non-coding sequence, a sequence encoding non-structural proteins, an internal ribosomal entry site (IRES) comprising an oligo(A) loop, a sequence encoding the protein to be expressed and a 3'-non-coding sequence in a suitable host cell and
- incubating this host cell with the expression construct to allow the protein to be expressed,
characterised in that the IRES carries a mutation in the oligo(A) loop.

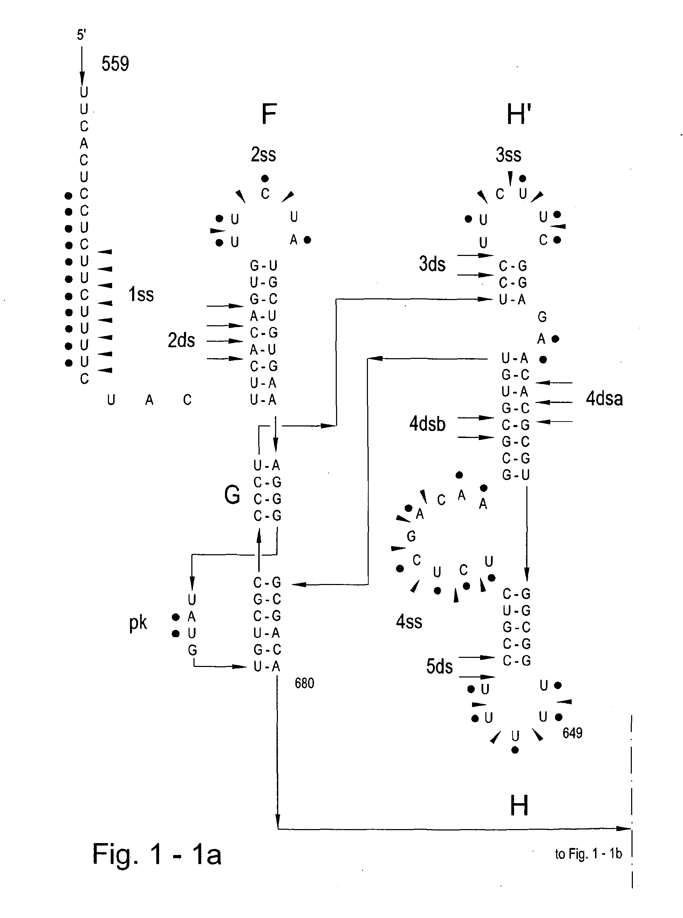

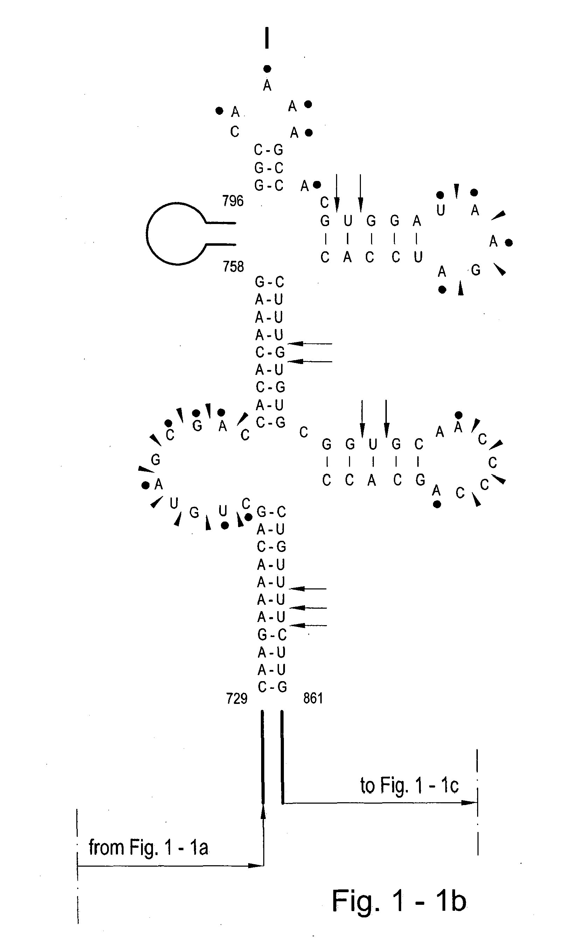

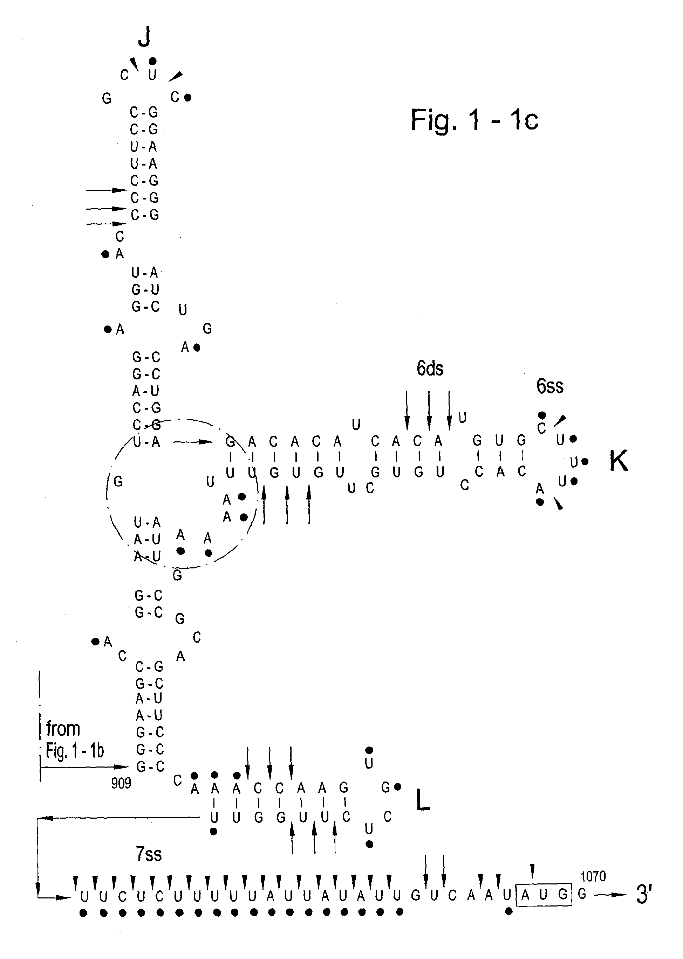

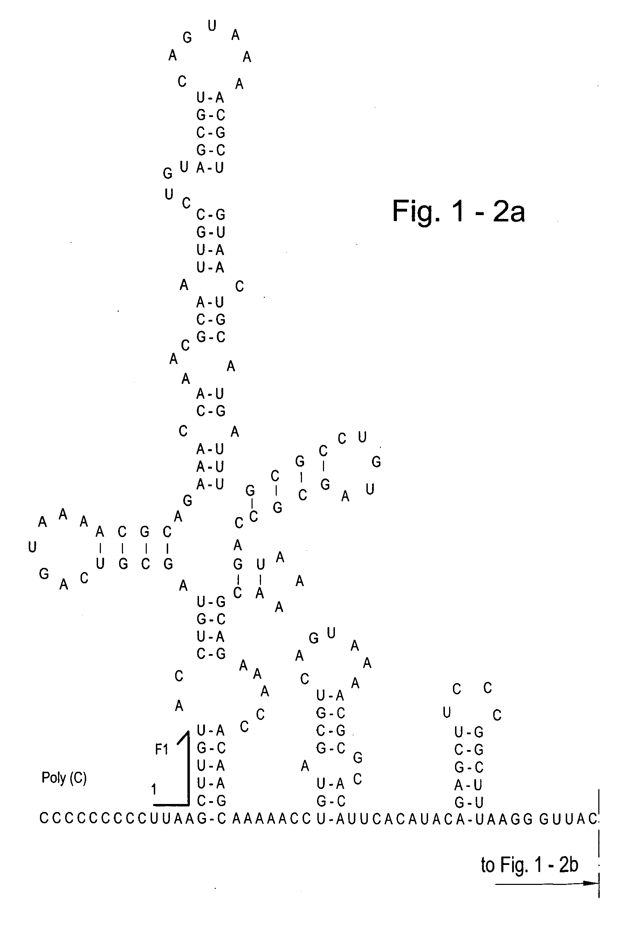

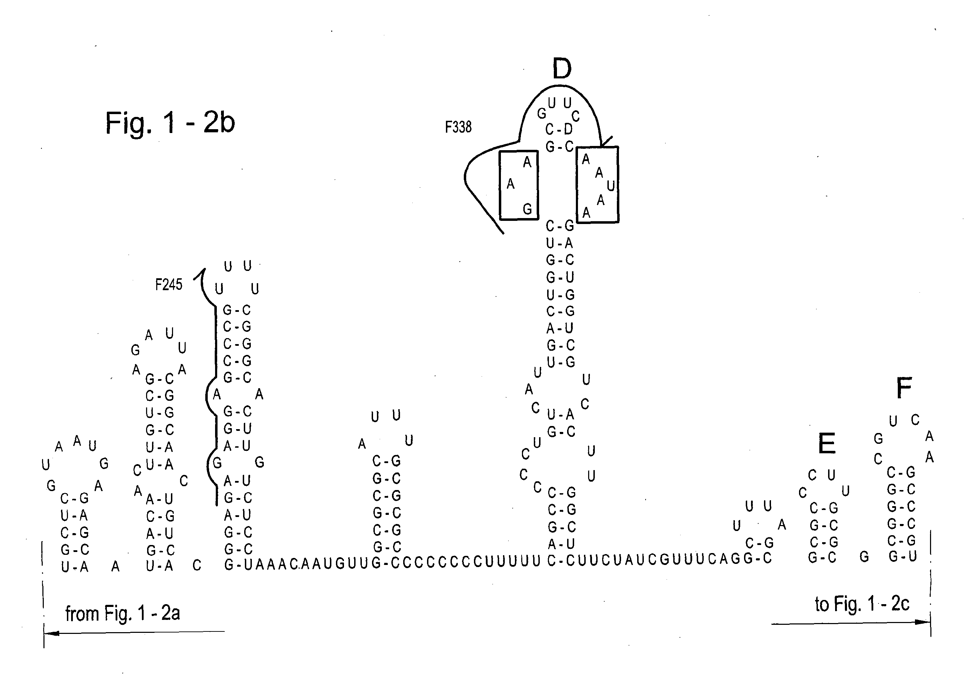

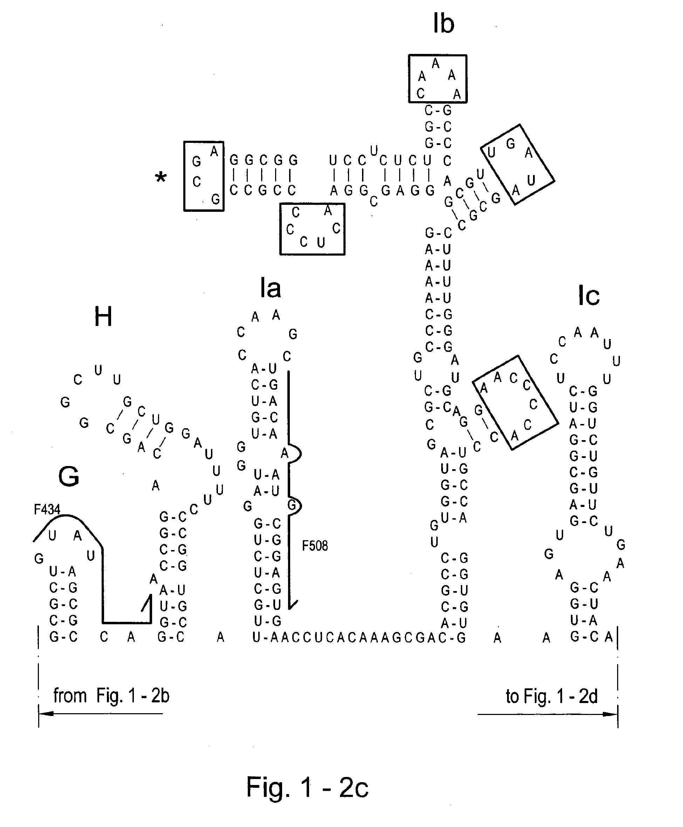

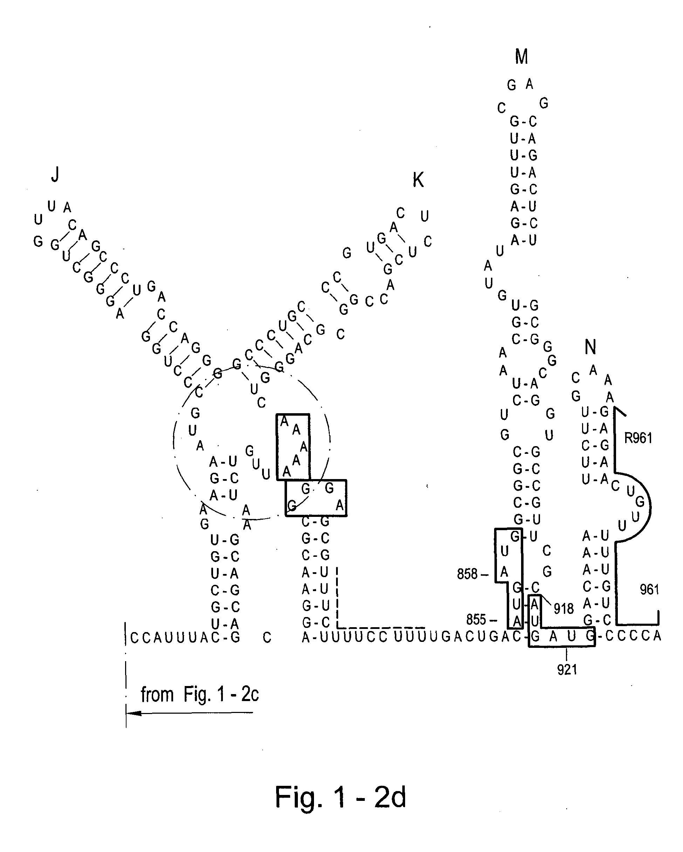

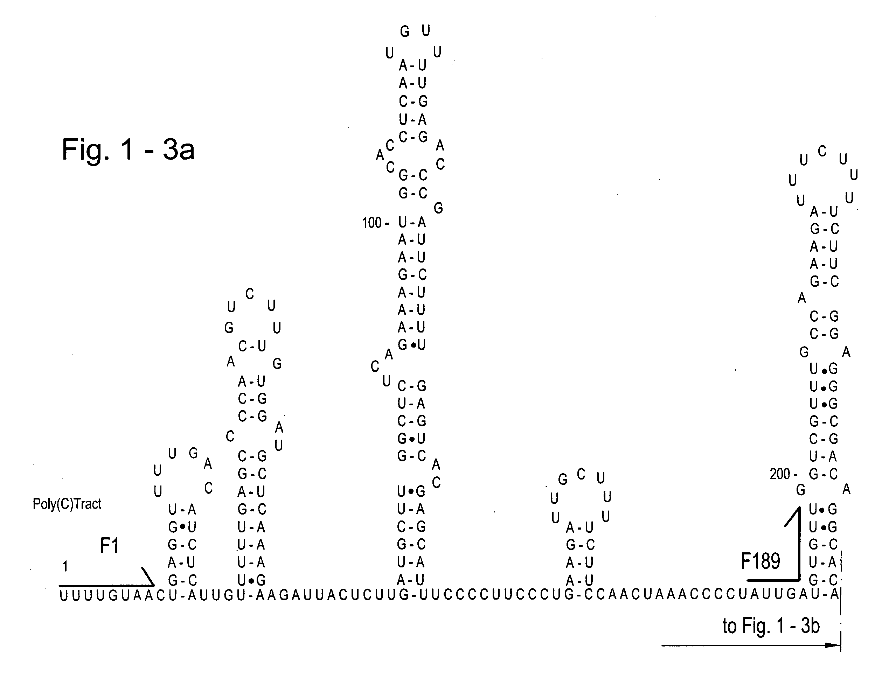

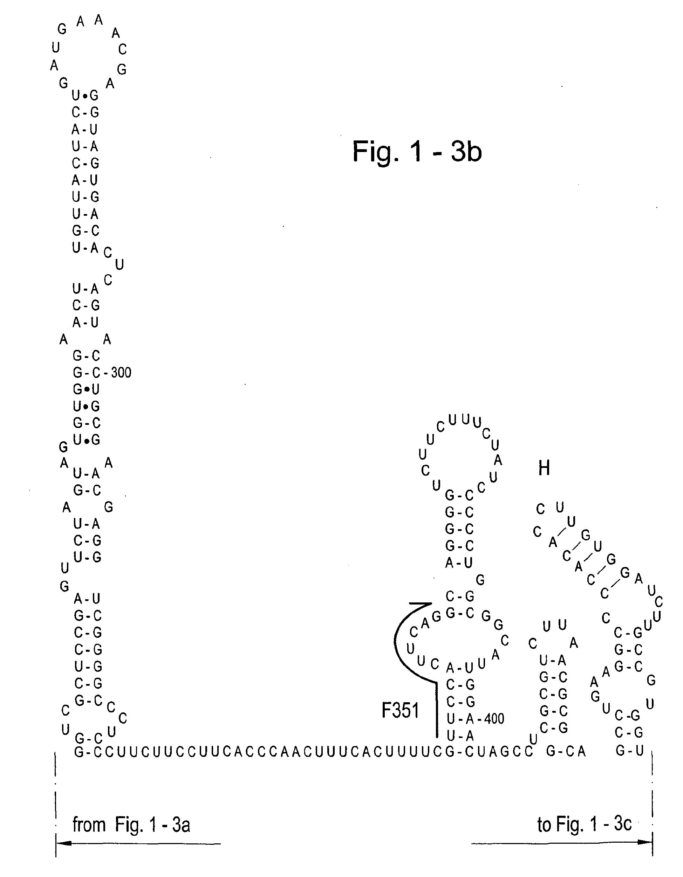

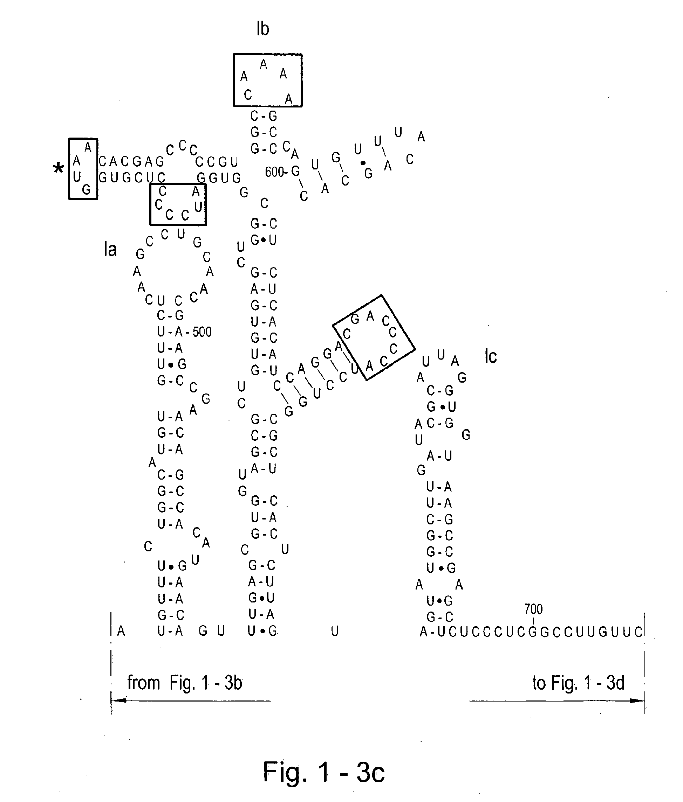

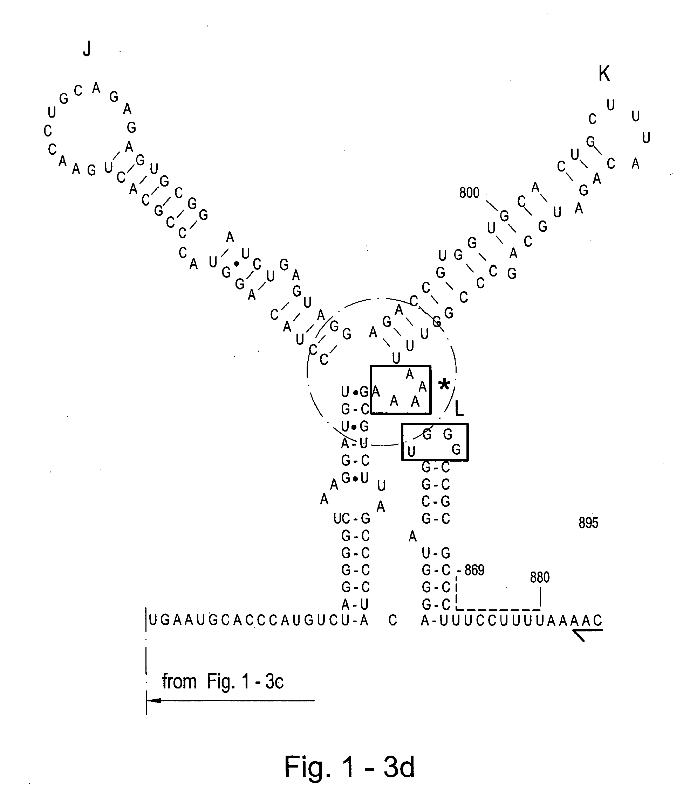

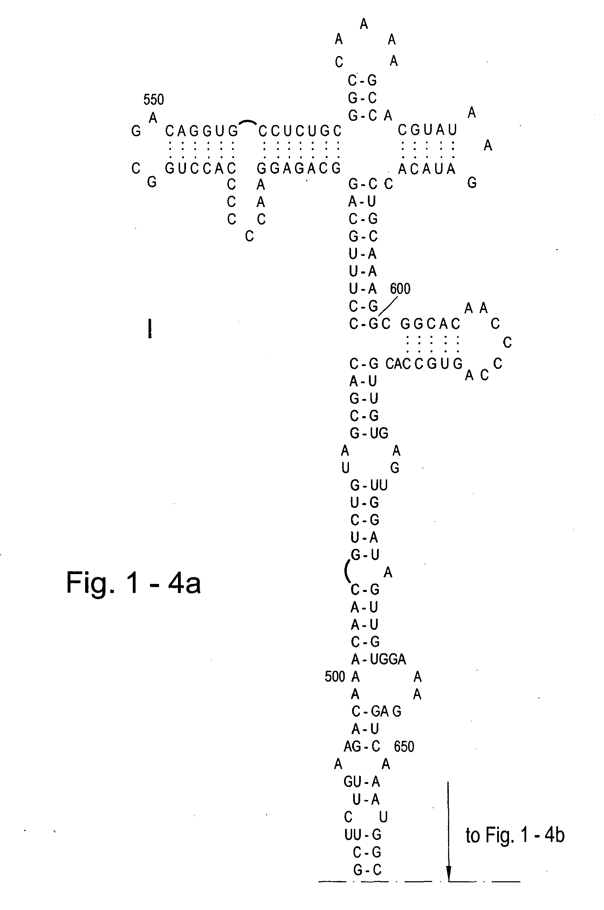

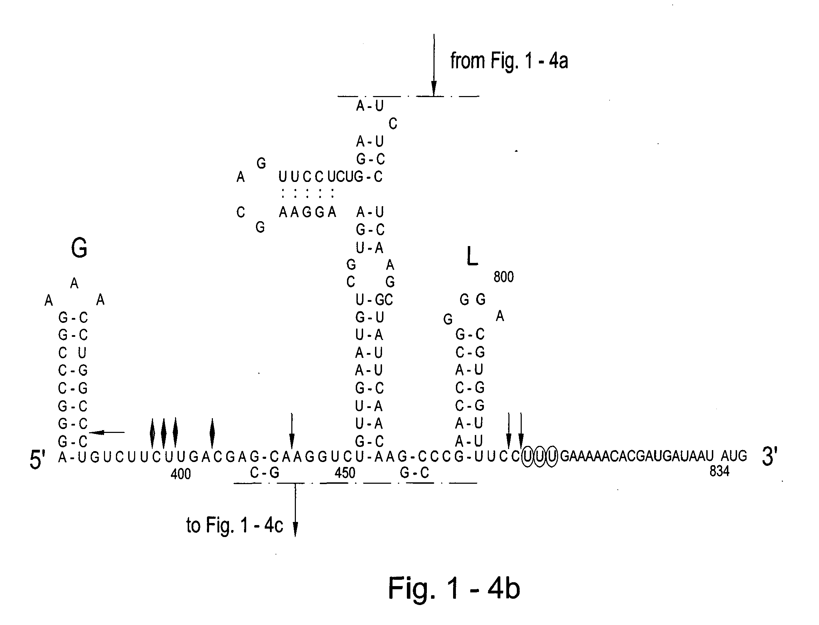

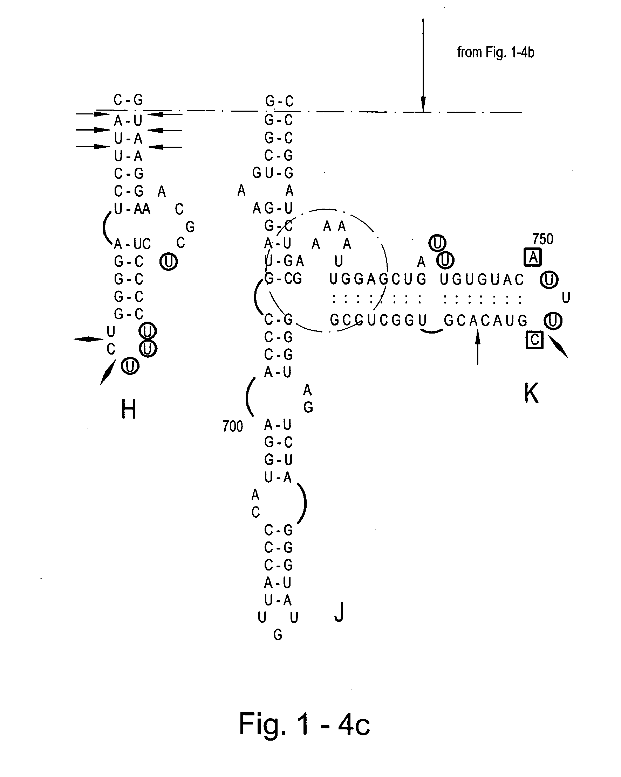

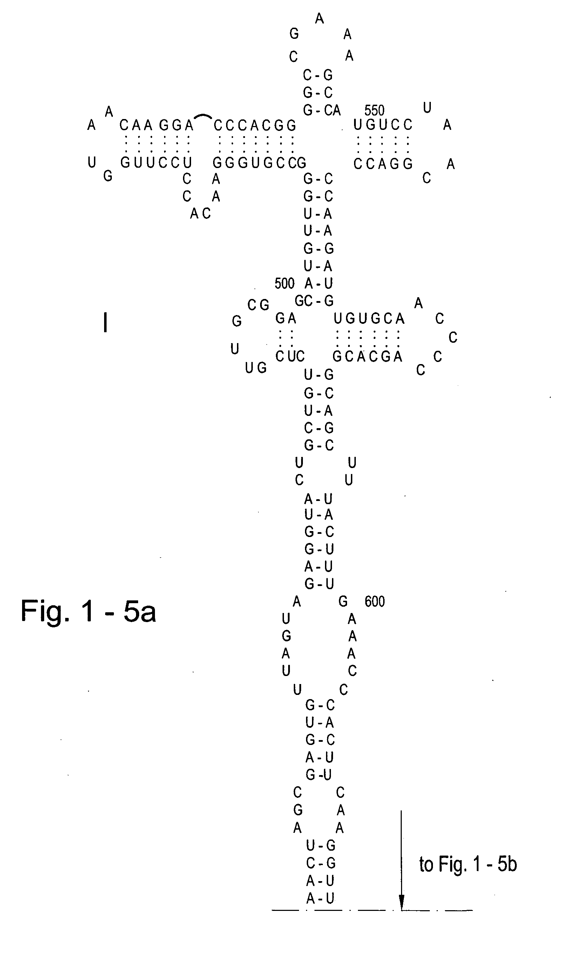

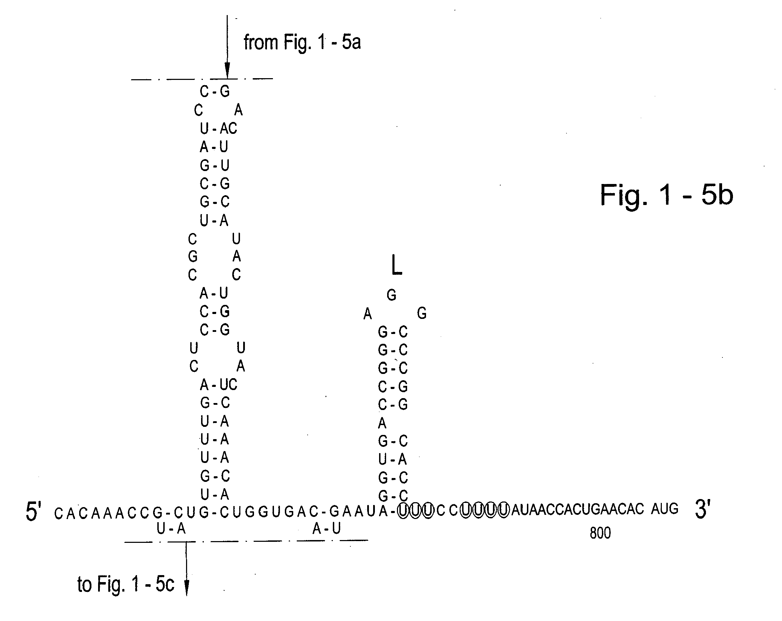

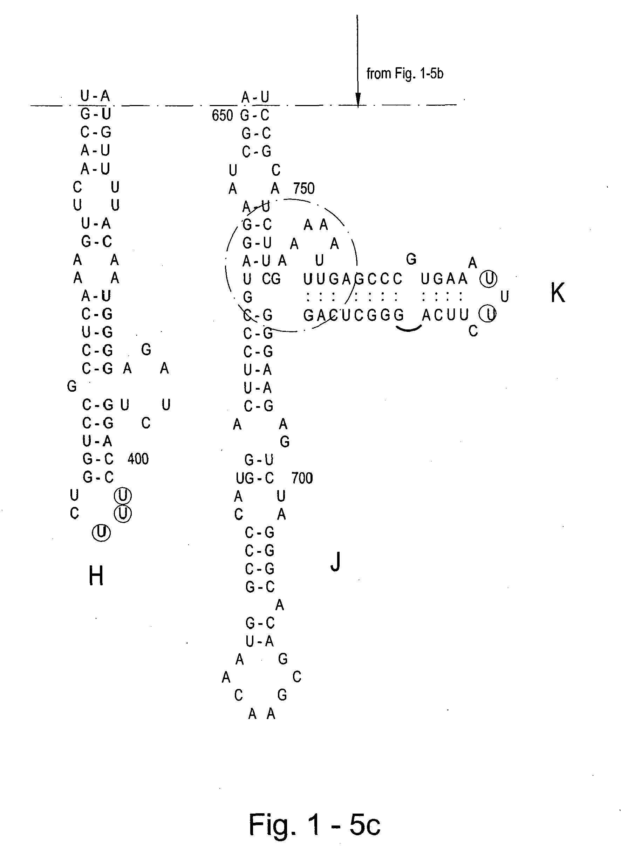

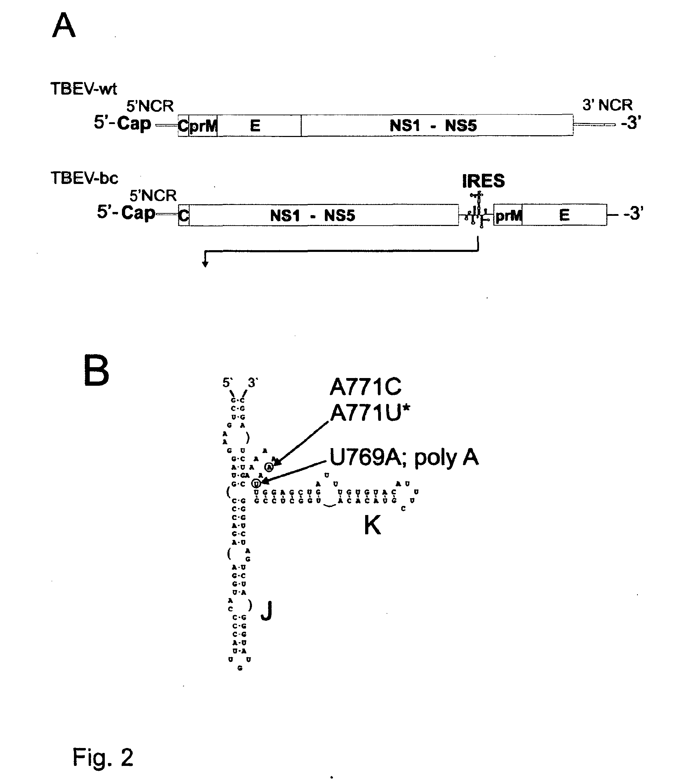

In the course of the present invention it was observed that certain mutations in the IRES (which are normally negatively affecting translational performance) surprisingly enhance the overall expression capabilities in a bicistronic flavivirus expression construct. The IRES mutations according to the present invention which improve expression performance are located in a specific region in the J-K stem loop structure, the so-called oligo(A) loop. This loop is an unpaired structure mainly consisting of A residues between the J and the K stem loop (often also referred to as "JK loop"). For example in the IRES of Encephalomyocarditis virus (EMCV), the oligo(A) loop is from U769 to A776 (see also: Fig. 2B (analogous: Figs.1-3)). Since EMCV IRES is one of the most prominently and most efficiently used IRES sequence, a preferred embodiment of the present invention is a construct wherein the oligo(A) loop is defined as consisting of base 769 to 776 of IRES of EMCV in the J-K stem loop.

The fact that overall expression performance in bicistronic flavivirus constructs can be improved by introducing mutations in the IRES is surprising for many reasons and probably unique to the flavivirus genus: First, it was known that expression levels are severely decreased by using bicistronic flavivirus constructs with IRES. Second, mutations affecting the oligo(A) loop have been described to have significantly decreased translational efficiency and expression performance. For example, Hoffman and Palmenberg have described various IRES mutations, some of them also affecting the oligo(A) loop (J. Virology 69 (1995), 4399-4406; J. Virology 70 (1996), 6425-6430; for example, a mutation of A772 to C (A772C) resulted in a more than 60 % reduction of in vivo expression; a deletion of the residues 768 to 771 reduced in vivo expression by more than 99 %). It was therefore completely unexpected that - in the overall process - the expression performance of the constructs according to the present invention was significantly improved by such IRES mutations.

Any IRES structure comprising an oligo(A) loop can be mutated in principle according to the present invention. A large number of different IRES structures has been described in the literature. In many of the known IRES structures the oligo(A) loop is present as a particular sequence element. This is a usually 6 to 8 residues long unpaired sequence of mainly A residues which is predicted to form a bulge between two adjacent stable stem-loop structures, which in the case of many IRES structures are referred to as J and K. For some IRES structures, in particular that of EMCV the functional importance of this loop has been demonstrated and is described in the literature. The oligo(A) loop and the adjacent J and K structures are shown in Fig. 1 for some important representatives of IRES structures, i.e. EMCV and FMDV (which are usually used in bicistronic expression vectors), but also other virus-derived IRES. The region wherein the oligo(A) loop is located is marked by a circle in this figure. Other IRES structures with analogous oligo(A) loops can be easily identified by preparing a secondary structure prediction with a conventional prediction algorithm (such as mFold), and identifying an unpaired sequence comprising e.g. 5 to 7 residues long stretch of (mainly) As between two stem-loop structures. According to the present invention mutations have been introduced that change one or several of the A residues of the oligo(A) loop and/or one of the (unpaired) nucleotides adjacent to this loop preferably by replacing the natural residue with another nucleotide. These point mutations (point mutations are a preferred embodiment of the present invention) can be combined with other mutations, preferably insertion mutations, in which additional A residues are inserted that increase the size of the oligo(A) loop. Specifically preferred are oligo(A) tails comprising 8 or more A residues. According to the present invention, the mutation can be a deletion, insertion or point mutation or combination of such mutations, including the combination of two or more point mutations.

Specifically preferred are mutations of the residue which is 5'-adjacent to the oligo(A) loop (usually a U residue in RNA, a T residue in the DNA template), specifically changing this U into an A residue, or the mutation of the second A residue of the oligo(A) loop, specifically changing this A residue into C or U.

One of the preferred general embodiments of the present invention relates to the expression of exogenous proteins with the flavivirus system according to the present invention. For doing so, the gene of a foreign protein has to be inserted into a given flavivirus genome. Preferably, this gene of a foreign protein encodes a non-flavivirus protein allowing expression of this non-flavivirus protein in the bicistronic system according to the present invention.

On the other hand, it is also preferred to use the present system for (enhanced) expression of flavivirus proteins. Preferably, the method according to the present invention employs a construct which comprises one or more structural protein(s) of the flavivirus. For example, the structural proteins of the given flavivirus vector can be used for the production of improved vaccines with the system according to the present invention. This can e.g. be done by expressing this structural protein(s) under the control of the mutated IRES.

Although the heterologous protein can also be expressed under the control of the CAP mechanism, it is preferred to express the foreign protein in an IRES dependent manner.

The mutations of the present invention have been optimised using a positive selection system to identify mutations with significantly enhanced expression efficiency (Orlinger et al, 2006). The high mutation rate of the viral replicase is responsible for a wide variety of random mutations. By passaging the virus in BHK-21 cells or by intracerebral inoculation in baby mice, improved mutants have been obtained. Preferred mutations according to the present invention are therefore selected from the group consisting of any base substitution of U769, A770, A771, A 772, an insertion of 1 to 50 nucleic acid residues at any base from U769 to A776, or combinations thereof. The numeration of all bases throughout the present specification are given for the EMCV IRES as a model; however, the numeration may be directly adapted to other IRES by the skilled man in the art. Preferred IRES according to the present invention are - besides EMCV IRES - IRES from Theilers Murine encephalomyelitis virus, Equine rhinitis virus A or B, Mengo virus or Foot-and-mouth disease virus (FMDV).

It is specifically preferred according to the present invention to use a mutation in the oligo(A) loop which is selected from the group consisting of U769A, A770U, A770C, A771U, A771C, A 772U, A772C, an insertion of 1 to 37 A residues at any base from U769 to A776, or combinations thereof.

The most preferred mutations according to the present invention are selected from the group consisting of U769A, A771U, A771C, or an insertion of 1 to 10 A residues at any base from U769 to A776.

With the constructs according to the present invention comprising the mutations in the IRES oligo(A) loop it was observed - as reported - that the translational efficiency of the IRES per se (i.e. not in context with the bicistronic flavivirus vector) is reduced, but the number of positively transfected cells with the bicistronic flavivirus vector and the flavivirus titre in the supernatant of infected cells is raised, the RNA replication is improved, the export of infectious bicistronic RNA and the expression rate of both cistrons in the context of the bicistronic flavivirus is enhanced. These results in combination lead to the - surprising - overall improvement of the expression performance of the bicistronic flavivirus construct according to the present invention.

Of course, it is clear to the skilled man in the art that the present constructs comprise or can comprise all further items known to be necessary or advantageous for bicistronic flavivirus expression constructs, such as start/stop codons, cyclisation sequences, compatible (coding/non-coding) element, etc.

Preferably, the constructs according to the present invention further comprise sequences encoding flavivirus surface proteins prM and E. It is then possible to produce sub-viral particles or virus-like particles. If additionally protein C is also provided, infectious viruses can be produced, which can be used for further propagating the construct to further host cells.

The host cell according to the present invention may be any host cell being capable of expressing the protein(s) of the expression construct according to the present invention. As already mentioned, flaviviruses have a rather broad host range. Nevertheless, the preferred host cells according to the present invention are those host cells for which successful expression of proteins using flaviviral vectors have already been described or even optimised. Preferably, an eukaryotic cell line is used as a host cell that is competent to effect transcription, translation and any post-transcriptional and/or post-translational processing or modification required for protein expression. Specifically preferred are those cells which are known to be efficiently applicable for the industrial production of proteins for human recombinant therapeutics. Therefore, the host cells according to the present invention are preferably selected from the group consisting of baby hamster kidney cells, Vero cells, Chinese hamster ovary cells, HeLa cells and Hep cells. Specific preferred cell lines are COS, Vero, CV-1, BHK21, HEK293, CHO, NIH 3T3, Jurkat, WEHI231, HeLa and B16 melanoma cells.

According to the present invention a wide range of gene therapeutic, prophylactic, therapeutic or diagnostic applications can be performed with the flavivirus constructs according to the present invention, especially for the production of antigens for prophylactic, therapeutic or diagnostic uses. For example, marker genes or resistance genes can be expressed by the constucts according to the present invention. Cells infected by these vectors can then easily be identified and permanent cell cultures can be generated which comprise such constructs. Cell cultures with cells comprising the constructs according to the present invention can be used for the production of gene products encoded by the present constructs, for example proteins for prophylactic or therapeutic immunising or diagnostic antigens. The gene products produced can be heterologous proteins or homologous proteins of the flavivirus. In a preferred embodiment, the construct produces the surface proteins prM and E and produces subviral or virus-like particles in the host cells. The bicistronic flaviviruses can be produced as infectious particles which may be used e.g. for immunising against the flavivirus or against the expressed heterologous gene product. Infectious or non-infectious constructs according to the present invention may be used for expressing a heterologous protein and - after inoculation in humans or animals (preferably vertebrate animals including domestic animals such as livestock and companion animals) - inducing an immune response against this protein. Alternatively, such constructs according to the present invention may be used for compensating gene defects or as tumour therapy or as a therapy for chronic diseases. Moreover, the present constructs may be used as research tools e.g. for translation and replication studies or for drug screening. The term "protein" as used herein shall have a broad meaning extending to short oligopeptides with a low number of amino acid residues (e.g. down to 5 amino acid residues).

Preferably, the protein to be expressed is selected from the group consisting of heterologeous (exogenous) polypeptides from pathogenic viruses, bacteria, yeast, fungi and protozoa, tumor associated antigens, "self" antigens or proteins with therapeutic activity, especially cytokines, hormones, bioactive peptides, soluble forms of membrane proteins, enzymes (preferably proteases, especially viral proteases, such as HIV or HCV protease), regulatory proteins, structural proteins, clotting factors, chemokines, erythropoietin or t-PA.

The present invention relates to a method for improving the yield of expressed protein in a bicistronic flavivirus expression system comprising
- providing a bicistronic flavivirus expression construct comprising a 5'-terminal CAP structure, a 5'-non-coding sequence, a sequence encoding non-structural proteins, an internal ribosomal entry site (IRES) comprising an oligo(A) loop, 3' non-coding sequences and a sequence encoding the protein to be expressed in a suitable host cell and
- incubating this host cell with the expression construct to allow the protein to be expressed,
characterised in that an IRES with a mutation in the oligo(A) loop is used.

Preferably, the order of the sequences is (from 5' to 3'):
- 5'-terminal CAP structure, 5'-non-coding sequence, sequence encoding non-structural proteins, internal ribosomal entry site (IRES) comprising the mutated oligo(A) loop, sequence encoding the protein to be expressed, 3' non-coding sequences; or
- 5'-terminal CAP structure, 5'-non-coding sequence, sequence encoding the protein to be expressed, internal ribosomal entry site (IRES) comprising the mutated oligo(A) loop, sequence encoding non-structural proteins, 3' non-coding sequences.

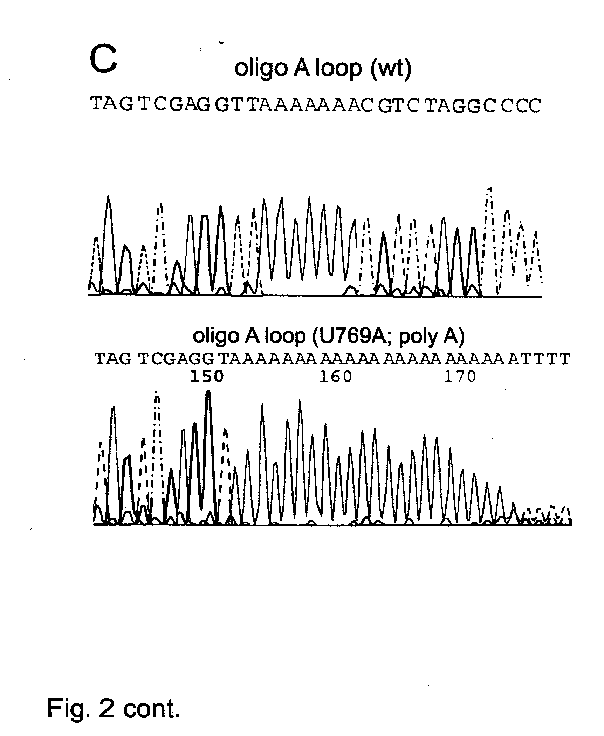

These two orders of sequences have proven to be the optimised way in bicistronic flavivirus constructs. For 5'-terminal CAP structure, 5'-non-coding sequence, sequence encoding non-structural proteins, and 3' non-coding sequences a variety of variations and mutations exists which are readily available for the skilled man in the art and which have essentially the same function in the present system as the wild type sequences. Preferably, flavivirus structural sequences are provided. These structural sequences are either controlled by the CAP mechanism or by the IRES. If a heterologous protein should be expressed by the construct according to the present invention, the structural sequences are preferably under the control of the CAP mechanism. In such a construct it is preferred to make use of the native order of sequences (5' non-coding sequences followed by the structural sequences and the non-structural sequences), then the IRES and the heterologous sequence, finally the 3' non-coding region. Preferably, the architecture of TBEV-bc (according to Fig. 2; Orlinger et al. (2006) is used (with the wild type IRES being replaced by the mutated IRES according to the present invention). Further variations are available to the skilled man in the art (see also e.g. WO 02/089840 A1).

The present invention relates to the use of an IRES with a mutation in the oligo(A) loop for improving the expression yield of a protein expressed in a flavivirus bicistronic expression system.

The present invention also relates to an IRES with a mutation selected from the group consisting of U769A, A771U, A771C, or an insertion of 1 to 10 A residues at any base from U769 to A776.

Expression systems according to the present invention can apply infectious or non-infectious vectors or constructs. Infectious vectors comprise all flavivirus genes and, if a foreign protein should be expressed, the gene for the foreign protein (or the genes, if more than one foreign protein should be expressed). These vectors assemble virus particles wherein the genomic RNA encoding the exogenous protein. These virus particles can infect further host cells. Non-infectious vectors ("replicons") can be produced by mutations in the structural proteins C, prM and E (Gehrke et al., J. Virology 77 (2003), 8924-8933). One or more of these genes are partially or completely deleted (or mutations are introduced into these genes which prevent formation of infectious virus particles). Such replicons can autonomously replicate their RNA but cannot pack this RNA into infectious virus particles and can therefore not infect further host cells. For introducing replicon RNA into a host cell, various methods are available in the art, including electroporation, lipofection, direct injection, etc.. Moreover, replicon RNA may be packed into so-called single-round infectious particles by using expression of structural proteins which complement the defect or missing structural proteins of the replicon in trans. Such particles can enter host cells and introduce the replicon but cannot induce further infection cycles (e.g. Gehrke et al., 2003).

According to another aspect, the present invention relates to a replication competent bicistronic flavivirus expression construct comprising a mutation in the oligo(A) loop of the IRES. This construct according to the present invention can be infectious or not and can include non-flavivirus proteins or not. A preferred embodiment of the construct according to the present invention is the use as expression vehicle for an exogenous protein. Accordingly, this preferred replication competent bicistronic flavivirus expression vector comprises a mutation in the oligo(A) loop of the IRES and a coding sequence for a non-flavivirus protein to be expressed 3' of (under the control of) this mutated IRES.

The most preferred position for the IRES element in the flavivirus genome is usually around or in the 3' non-coding region. This region consists of a structurally and functionally essential and conserved 3' terminal region and a more flexible, less conserved region of varying length immediately after the stop codon of the reading frame.

For example, for the TBE virus, these regions have been termed as variable region and core element. It has been shown that the variable region of the 3' non-coding region may be replaced by an IRES expression cassette. In various flaviviruses it was shown that such a cassette can be inserted immediately after the 3' end of the first cistron under preservation of (almost) the whole 3' non-coding region. It is, however, also known that the IRES element may be integrated at other sites in the flavivirus genome and to put various structural or non-structural proteins under the control of the heterologous IRES element.

The heterologous gene(s) in flavivirus expression systems can be provided in various modes in the present construct: For example, the heterologous protein may be expressed under the control of the CAP mechanism or under the control of the IRES.

The present invention also provides an infectious recombinant bicistronic flavivirus comprising an IRES, characterised in that the IRES comprises a mutation in the oligo(A) loop.

The flavivirus to be used according to the present invention can be any member of the genus, for example tick-borne-viruses (mammalian tick-borne virus group, seabird tick-borne virus group), mosquito-borne viruses (Aroa virus group, Dengue virus group, Japanese encephalitis group, Kokobera virus group, Ntaya virus group, Spondweni virus group, Yellow fever virus group), or flaviviruses with no known arthropod vector (Entebbe bat virus group, Modoc virus group, Rio Bravo virus group). Examples for such flaviviruses are Gadgets Gully virus, Kyasanur forest disease virus, Langat virus, Louping ill virus (British, Irish, Spanish, Turkish subtype), Omsk hemorrhagic fever virus, Powassan virus, Royal Farm virus, Karshi virus, tick-borne encephalitis virus (European, Far Eastern, Siberian subtype); seabird tick-borne virus, such as Kadam virus, Meaban virus, Saumarez Reef virus, Tyuleniy virus; Aroa virus, Bussuquara virus, Iguape virus, Naranjal virus, Dengue virus (1, 2, 3, 4), Kedougou virus, Cacipacore virus, Japanese encephalitis virus, Koutango virus, Alfuy virus, Murray Valley encephalitis virus, St. Louis encephalitis virus, Usutu virus, West Nile virus, Kunjin virus, Yaounde virus, Kokobera virus, Stratford virus, Ntaya virus, Bagaza virus, Ilheus virus, Rocio virus, Israel turkey meningoencephalomyelitis virus, Ntaya virus, Tembusu virus, Spondweni virus, Zika virus, Banzi virus, Bouboui virus, Edge Hill virus, Jugra virus, Potiskum virus, Saboya virus, Sepik virus, Uganda S virus, Wesselsbron virus, Yellow fever virus, Entebbe bat virus, Sokoluk virus, Yokose virus, Apoi virus, Cowbone Ridge virus, Jutiapa virus, Modoc virus, Sal Vieja virus, San Perlita virus, Bukalasa bat virus, Carey Island virus, Dakar bat virus, Montana myotis leukoencephalitis virus, Phnom Penh bat virus, Batu Cave virus, Rio Bravo virus, Cell fusing agent virus and Tamana bat virus.

The preferred viruses to be used according to the present invention are the viruses that are most prominently used in current industrial approaches (i.e. for which efficient expression systems are already established). Accordingly, it it preferred to use yellow fever virus (YFV), Dengue virus serotypes 1 to 4, Japanese encephalitis virus, West Nile virus, Kunjin virus or Tick-borne encephalitis virus for the present invention.

The present invention further relates to the host cells comprising a flavivirus or a construct according to the present invention. The host cells may be cultured and used for gene expression for industrially producing the protein to be expressed.

The present invention also relates to a nucleic acid molecule encoding a replication competent bicistronic flavivirus expression construct comprising a mutation in the oligo(A) loop of the IRES.

According to another aspect, the present invention relates to the use of a nucleic acid according to the present invention for the manufacture of a pharmaceutical composition, especially immunotherapeutic compositions and vaccines. A particular aspect of the invention relates to use of the flaviviral expression construct and expression system as a vaccine delivery system. However, the invention more broadly provides a pharmaceutical composition of the nucleic acid not limited to use in vaccine delivery, but inclusive of immunotherapeutic compositions and vaccines that may comprise: (i) virus like (subviral) particles produced by the expression system of the invention ; (ii) an RNA transcribed from a DNA expression construct of the invention; or (iii) a plasmid DNA expression construct of the invention directing transcription of replicating RNA in vivo.

The pharmaceutical composition may further comprise a pharmaceutically acceptable carrier, diluent or excipient, such as a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water. A useful reference describing pharmaceutically acceptable carriers, diluents and excipients is Remington's Pharmaceutical Sciences (Mack Publishing Co. N. J. USA, 1991). Such compositions may be delivered for the purposes of generating immunity, preferably protective immunity, to pathogens such as viruses, bacteria, protozoan parasites and invertebrate parasites although without limitation thereto. In an alternative embodiment, immunotherapeutic treatment of cancers, such as melanoma, is contemplated by the present invention.

Any safe route of administration may be employed for providing a patient with the composition of the invention. For example, oral, rectal, parenteral, sublingual, buccal, intra-venous, intra-articular, intra-muscular, intra-dermal, subcutaneous, inhalational, intra-ocular, intra-peritoneal, intra-cerebroventricular, transdermal and the like may be employed. Intra-muscular and subcutaneous injection is appropriate, for example, for administration of immunotherapeutic compositions, proteinaceous vaccines and nucleic acid vaccines. Dosage forms include tablets, dispersions, suspensions, injections, solutions, syrups, troches, capsules, suppositories, aerosols, transdermal patches and the like. These dosage forms may also include injecting or implanting controlled releasing devices designed specifically for this purpose or other forms of implants modified to act additionally in this fashion. Controlled release of the therapeutic agent may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

Pharmaceutical compositions of the present invention suitable for oral or parenteral administration may be presented as discrete units such as capsules, sachets or tablets each containing a pre-determined amount of one or more therapeutic agents of the invention, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association one or more agents as described above with the carrier which constitutes one or more necessary ingredients. In general, the compositions are prepared by uniformly and intimately admixing the agents of the invention with liquid carriers or finely divided solid carriers or both, and then, if necessary, shaping the product into the desired presentation.

The above compositions may be administered in a manner compatible with the dosage formulation, and in such amount as is pharmaceutically-effective. The dose administered to a patient should be sufficient to effect a beneficial response in a patient over an appropriate period of time. The quantity of agent(s) to be administered may depend on the subject to be treated inclusive of the age, sex, weight and general health condition thereof, factors that will depend on the judgement of the practitioner. Immunotherapeutic compositions of the invention may be used to prophylactically or therapeutically immunise animals such as humans.

The present invention is further illustrated by the following examples and the figures, yet without being restricted thereto.
Fig. 1 shows IRES from Theilers Murine encephalomyelitis virus, Equine rhinits virus A or B, EMCV or Foot-and-mouth disease virus (FMDV).
FIG. 2. TBEV-bc genome and adaptive mutants according to the present invention. A: Genome organization of wild-type TBEV and bicistronic TBEV-bc. The region encoding structural proteins C, prM and E are shown in dark grey boxes and the region encoding non-structural proteins NS1 - NS5 is indicated in light grey boxes. B: Sequence and secondary structure of the EMCV IRES J-K stem loop showing the positions of selected mutations in the oligo A loop region. The asterisk (*) indicates a mutation that was independently isolated from suckling mouse brain. C. Electropherograms of sequencing reactions showing the sequence of the original oligo A loop and a representative U769A/ poly A mutant with multiple adenine insertions.
FIG. 3 shows the IRES translation capacity measured on day 1 after electroporation (wild type/769(45A loop) /769(26As)/769(19As)/771C; y-axis: % translation of wild type EMCV IRES)
FIG. 4 shows wild type (left) and mutation according to the present invention (right) in IF-positive cells
FIG. 5 shows titre determination of wild type (left) and mutated IRES according to the present invention (right); titre log on the y-axis
FIG. 6A shows RNA copies per 2000 cells (18 h after electroporation); Fig. 6B shows luciferase expression (14 h after electroporation) in relative light units on the y-axis; each of wild type (left) and mutated IRES according to the present invention (right)
Fig. 7 shows the amount of exported RNA (log on y-axis) of wild type (left) and mutated IRES according to the present invention (right)
Fig. 8A shows CAP protein expression; Fig. 8B shows IRES protein expression; each of wild type (left) and mutated IRES according to the present invention (right)(fluorescence intensity on y-axis)

### EXAMPLES:

### Materials and Methods

### Virus and cells:

All of the viruses and genomic constructs used in this study were based on the TBE virus prototype strain Neudoerfl (GenBank accession number U27495), and an infectious clone of this strain (Mandl et al., J. Gen. Virol. 78 (1997), 1049-1057) was used as the monocistronic wild-type control in all experiments.

BHK-21 cells (ATCC CCL10) were grown at 37°C and 5% CO₂ in Eagle's minimal essential medium (Sigma) supplemented with 5% fetal calf serum (FCS), 1% glutamine, and 0.5% neomycin. After transfection, the growth medium was replaced by a maintenance medium consisting of minimal essential medium with the FCS concentration lowered to 1% and buffered with 15 mM HEPES.

COS-1 cells (ATCC CRL 1650) were grown at 37°C and 5% CO₂ in Dulbecco's modified Eagle medium (DMEM) containing 10% FCS, 4 mM glutamine, 100 U penicillin, and 100 µg streptomycin per ml. After transfection, the medium was replaced with the same medium with the FCS concentration reduced to 1%.

### Virus titration:

Supernatants of infected or RNA-transfected cells were sequentially diluted in either log10 or 0.5-log10 steps, depending on the experiment, and 200 µl of each dilution was applied to fresh BHK-21 cells grown on coverslips in a 24-well cluster plate. Three days later, cells were fixed and permeabilised, and an indirect immufluorescence assay was carried out as described previously using polyclonal rabbit antisera (Kofler et al., J.Virology 76 (2002), 3534-3543). The endpoint dilution was defined as the last dilution at which positive fluorescent cells were detected.

### Selection of mutants:

Genomic-length TBEV-bc and TBEV-wt RNA were synthesized by in vitro transcription of plasmids pTNd/bc and pTBEV-c, respectively, as described previously (Mandl et al., J. Gen. Virol. 78 (1997), 1049-1057); Orlinger et al., 2006). After RNA synthesis, template DNA was digested by incubating with DNaseI for 15 min at 37°C, and the RNA was purified using an RNeasy Mini Kit (QIAGEN). The quality of the RNA was checked by formalin-denaturing agarose gel electrophoresis, and the concentration was determined spectrophotometrically (Kofler et al., J. Virology 80 (2006), 4099-4113).

BHK-21 cells were transfected by electroporation with the RNA as described previously (Mandl et al., 1997; Orlinger et al., 2006) and distributed into each well of a 24-well plate. After 16 h, the cells were washed twice with 0.5 ml of maintenance medium per well and then allowed to grow in 1 ml of maintenance medium for another 6 days, after which the supernatants were collected and cleared by low-speed centrifugation at 4°C. Fresh BHK-21 cells in another 24-well plate were then inoculated with 0.2 of cleared supernatant from the previous passage. After 90 min, the inoculum was removed, the cells were washed twice as above, and these cells were likewise allowed to grow for 6 days in 1 ml of fresh medium. This procedure was repeated a total of 6 times with supernatants from all wells that showed strong CPE, and the infectious titre was checked after each passage using the endpoint titration assay.

Cell supernatants containing variants that had exhibited a significant increase in virus titre during the sequential passaging steps were then used to infect fresh BHK-21 cells in 25-cm² tissue culture flasks. These infected cells were then harvested by trypsinisation 4 days after infection, and cytoplasmic RNA was isolated using an RNeasy Mini Kit (Qiagen). This RNA was reversed transcribed using a cDNA Synthesis Kit (Roche), and selected regions of this cDNA were amplified by PCR and sequenced using the appropriate primers.

### RNA export assay:

Aliquots of 140 µl of supernatant from transfected cells were cleared by low-speed centrifugation, and the viral RNA was isolated using a QIAamp viral RNA Mini Kit according to the manufacturer's instructions. Ten µl of the isolated RNA was used as a template for cDNA synthesis using an iScript cDNA Synthesis Kit (BioRad) according to the manufacturer's protocol. Half of this material was then used directly for quantitative real-time PCR analysis (PE Applied Biolsystems). The original RNA concentration of each sample was determined using a standard curve in which serial 10-fold dilutions of an RNA preparation of known concentration were subjected to the same amplification steps as the samples.

### Cloning techniques and construction of mutants:

Selected IRES variants were cloned into the commercially available expression vector pIRES2-eGFP (Clontech) using the unique restriction sites for SacII and BstXI that flank the IRES in this plasmid. cDNA from the selected variants was amplified using an Advantage-HF 2 PCR Kit (BD Bioscience) with specific primers containing the appropriate restriction enzyme recognition sites, and after cutting the vector and insert with these enzymes, these were ligated together and introduced by electro-transformation into Escherichia coli strain HB101.

The construction and characterization of the full-length plasmid clone, pTNd/bc, which was used to generate the bicistronic RNA genome of TBEV-bc and its derivatives, was described in a previous study (Orlinger et al., 2006). The genomic RNA generated from this construct lacks the region encoding the prM and E proteins in the open reading frame encoding the viral polyprotein but retains the portions encoding the capsid protein and all of the viral non-structural proteins in the natural CAP-dependent cistron. An EMCV IRES inserted into a variable region of the 3'-non-coding region (3'-NCR) followed by the region encoding the prM and E glycoproteins allows separate expression of these proteins (Gehrke et al., 2005).

IRES mutations were first introduced into an intermediate plasmid clone containing only the IRES cistron, pIRES2-prME, which had been constructed previously (Orlinger et al., 2006). pIRES2-prME vectors carrying IRES mutations were assembled in three-fragment ligations utilizing (i) individual IRES fragments excised by BstX I and Sac II restriction enzymes from mutant pIRES2-eGFP vectors, (ii) BstXI- and SacII-digested pIRES2-prME (lacking the IRES and the 5' part of the contiguously encoded prME sequence) vector backbone and (iii) the BstXI-SacII-excised fragment encoding the 5' part of the prME coding sequence. TBEV-bc constructs carrying mutations only in the IRES element were constructed by exchanging a fragment from the corresponding pIRES2-prME mutant, using a unique Sac II restriction site directly upstream of the IRES element and a unique SnaBI site in the protein E coding region.

### Measurement of NS1 and E protein expression:

The relative amounts of NS1 and E protein in BHK-21 cells after transfection were determined by FACS analysis as in a previous study (Kofler et al., J. Virology 76 (2002), 3534-3443). Cells were transfected by electroporation with the appropriate genomic RNA and transferred to 25-cm² tissue culture flasks. The medium was replaced 20 h after transfection with maintenance medium and allowed to grow for different lengths of time until harvest. At each time point, cells were harvested by trypsinisation and washed three times in phosphate-buffered saline (PBS) containing 2% bovine serum albumin (BSA). Then, cells were counted in a Casy TT cell counter (Schärfe Systems) and 200,000 cells were fixed and permeabilised using the provided Lysing and Perm2 solutions (BD) diluted 1:10 in PBS. After each reaction, cells were washed twice with PBS containing 0.5% BSA. After permeabilisation, cells were split into two halves and incubated for 30 min at 37°C with either a monoclonal anti-E antibody (B2) (Heinz et al., J. Biol. Stand. 14 (1986), 133-141) or a monoclonal anti-NS1 antibody (6E11) (Iacono-Connors et al., Virus Res. 43 (1996), 125-136), followed by two consecutive washing steps. Cells were then stained with fluorescein-isocyanate-conjugated anti-mouse antibodies (Jackson Immuno Research Laboratories) for 30 min at 37°C. Two further washing steps served to remove non-specifically bound antibodies, and stained cells were resuspended in 200 µl PBS containing 0.5% BSA.

A FACSCalibur flow cytometer (Becton Dickinson) (15-mW argon laser, 488 nm) with a 530/30 bandpass filter (FL-1) was used to quantify fluorescence of the stained cells. The main cell population was gated and 10,000 cells counted per sample. Analysis of the samples was carried out using CellQuestPro software to determine the fluorescence intensity of the brightest 10% of the cells to correct for differences in electroporation efficiency.

### Results

### Selection of adaptive mutations in BHK-21 cells:

The altered genetic organization of the artificial bicistronic TBEV construct, TBEV-bc, is shown in comparison to the normal, monocistronic wild-type genome (TBEV-wt) in Fig. 2A. In TBEV-bc, the prM and E glycoproteins are no longer expressed as part of the normal CAP-dependent cistron at the 5' end of the genome, but instead, this region is translated separately under the control of an inserted ECMV IRES element at the 3' end of the genome. All of the other viral proteins, i.e. capsid protein (C) and the viral non-structural proteins (NS1-NS5), are still translated as part of the original CAP cistron. This altered genome arrangement yields viruses that are viable but replicate considerably less efficiently than wild-type virus (Orlinger et al., 2006).

In order to establish a basis for optimizing the efficiency of bicistronic flavivirus vectors, it was attempted to generate adaptive mutations in TBEV-bc by repeated passage in cell culture. This was done by transfecting BHK-21 cells with in vitro-synthesized full-length genomic TBEV-bc RNA, collecting the cell supernatant 6 days later, and then using this supernatant as the initial inoculum for multiple cycles of serial passage in fresh BHK-21 cells. An equal amount of infectious RNA from a wild-type clone (TBEV-wt) was used as a control.

After the first passage it was observed that the infected cells in about 20% of the wells displayed morphological changes that were visible in the light microscope. When the supernatants from all 24 wells were then used to infect fresh BHK-21 cells, the same pattern was observed again, with the same samples causing altered cell morphology. These samples were therefore used for all further passages.

Repeated passage of TBEV-bc resulted in sequential increases in infectious titre, indicating that variants were being selected. The endpoint titres generally increased from about 10¹-10² in the first passage to about 10⁵ in the sixth passage. The titre of the wild-type control increased from about 10⁵ to 10⁷ after the first passage and then remained constant after that.

To identify which mutations had been selected, supernatants from the sixth passage were used to infect fresh BHK-21 cells, and viral RNA was collected from the cytoplasm of these cells and reverse transcribed into cDNA. Nucleotide sequencing of this cDNA revealed that these mutants contained nucleotide substitutions and insertions in a limited region of the inserted IRES element (Fig 2B). The complete genomes of two of the mutants were sequenced, and in both cases, no other mutations were found in any other region of the genome (except (expected) mutations in protein E which are known to provide adaptation to BHK21 cells), indicating that these specific mutations were indeed the ones responsible for the increased titres.

The mutations in the EMCV IRES all occurred in an important functional region, the conserved oligo(A) loop, which forms a bulge in the J-K stem-loop structure (Fig 2B). This loop is directly involved in binding the eukaryotic translation initiation factor 4G (eIF4G) and therefore plays a critical role in CAP-independent translation. One of the selected mutations was an A-to-C change at the position in the IRES corresponding to nuclotide 771 of the EMCV genome (GenBank accession no. M81861), which is the second A of the loop (Fig. 2B).

Another group of mutations, U769A/polyA, resulted in a change in the last U preceding the loop to an A, accompanied by the insertion of multiple adenosine residues into the loop (Fig. 2C). Direct sequencing of the cDNA showed that the number of A's was variable within the RNA population, resulting in a mixture of lengths. By subcloning this region into a pIRES2-eGFP expression vector and sequencing 10 different clones, it was found that the number of additional A's added to the loop ranged from 11 to 37, thus increasing the original loop size from 7 A's to as many as 45 (Table 1).

**TABLE 1. Adenine insertions in oligo A loop**

| Mutant | Number of inserted A residues | Nucleotide at position 769 | total number of A residues |
|---|---|---|---|
| TBEV-bc | 0 | U | 7 |
| 1 | 11 | A | 19 |
| 2 | 13 | A | 21 |
| 3 | 15 | A | 23 |
| 4 | 16 | A | 24 |
| 5 | 16 | A | 24 |
| 6 | 17 | A | 25 |
| 7 | 18 | A | 26 |
| 8 | 19 | A | 27 |
| 9 | 23 | A | 31 |
| 10 | 37 | A | 45 |

None of the 10 mutants was predicted by the RNA secondary structure prediction software mfold (Zuker, Nucleic Acids Res. 31 (2003), 3406-3415) to change the overall RNA fold of the IRES; they only increased the size of the oligo(A) loop. It is clear that the population of mutants also included variants with other numbers of A's, possibly forming a continuum. Furthermore, since it is possible that at least some of the additional A's were added by slippage of Taq polymerase during the amplification procedure rather than by the viral polymerase during replication, it cannot be concluded that a particular number of A's had actually been selected in cell culture. However, relatively large insertions of A's at this position are surprisingly well tolerated and allow viable progeny to be produced.

### Selection of an A771U mutation in mouse brain:

A bicistronic derivative of TBEV-bc (TBEV-bcYF) in which the region coding for the second transmembrane segment of the E protein was produced by replacement with the corresponding region from another flavivirus, yellow fever virus. Although this mutant was severely impaired and failed to cause a lethal infection in 22 of 23 suckling mice, one mouse in this experiment developed encephalitis and died. Sequence analysis showed that this sequence contained an A-to-U substitution at position 771 in the IRES - exactly the same nucleotide position where an A-to-C substitution had occurred in BHK-21-cell-passaged virus (Fig. 2B). The selection of a change at nucleotide 771 in mouse brain as well as in cell culture provides corroborating evidence that replacing the adenine residue at this position in the bicistronic genome increases viral fitness.

### Effect of IRES mutations on specific infectivity and virus production:

All of the mutants that were generated in BHK-21 cells contained changes in the IRES. In order to study the individual effect of the mutations, they were cloned back into the original TBEV-bc plasmid construct and the new clones were used to make infectious RNA in vitro for transfection.

In the first set of experiments, it was compared: (i) TBEV-bc(IRES-A771C), which has only a single change in the IRES, (ii) TBEV-bc, which is the bicistronic parent construct without any adaptive mutations, and (iii) TBEV-wt, the normal, monocistronic wild-type TBE virus infectious clone.

Cells were transfected with equal amounts of full-length infectious RNA corresponding to each of these variants, and infectivity was determined on day 6 after transfection by the endpoint titration assay. All of the mutants had a higher infectious titre than the original bicistronic construct. The A771C IRES mutation by itself resulted in an approximately 2-log increase in titre. This indicates that the mutations according to the present invention are advantageous for viral growth.

The increase in viral titre could be a reflection of: (i) an increase in the total number of infectious particles that were produced and released into the cell supernatant, (ii) an increase in the specific activity of the individual particles, or (iii) a combination of both effects. To distinguish between these possibilities, the level of secretion of virus particles was examined by measuring the amount of viral RNA released from the cells, as well as the specific infectivity of these particles, by correlating the total amount of extracellular viral RNA with the infectious titre for each mutant.

To analyse particle secretion, BHK-21 cells were transfected with equal amounts of viral RNA, and cell supernatants were collected 6 days after transfection. The total amount of viral RNA that was released into the cell supernatants was measured by real-time PCR. This experiment demonstrated that the mutants containing the A771C mutation in the IRES showed higher levels of extracellular viral RNA. In the case of the A771C mutation the amount of virus was about 20-fold higher than with TBEV-bc and reached about half of the level achieved with a normal monocistronic wild-type construct. This shows that the single nucleotide change in the IRES was responsible for an increase in the overall rate of virus particle production.

To determine the specific infectivity of the particles produced by each construct, BHK-21 cells were transfected with the appropriate viral RNA, and one aliquot of the infectious material in each cell supernatant was used to determine the titre by endpoint dilution assay, while another aliquot was used to measure the amount of genomic RNA by the real-time PCR method. This allowed the specific infectivity of each sample to be expressed as viral RNA equivalents per infectious unit (see Materials and Methods).

All of the mutants displayed a decreased RNA-to-endpoint ratio, i.e. an increased specific infectivity (A771C IRES mutation: 5-7-fold).

### Effect of IRES mutations on reporter gene expression:

Next, a closer look was taken at the effect of the A771C mutation on the function of the IRES itself, i.e. its efficiency as an initiation site for translation. To do this, the A771C mutation was introduced into the reporter construct pIRES2-eGFP, which allows translation efficiency to be monitored by measuring expression of enhanced green fluorescent protein. The mutant and parent reporter plasmids were introduced into COS-1 cells by electroporation, and cellular fluorescence due to eGFP expression was measured by FACS at 24-hour intervals for 3 days. The modified IRES containing the A771C mutation was less efficient than the parent construct, with fluorescence levels reduced by 40-50%. This result was somewhat unexpected considering that this mutation, in the context of the bicistronic construct, had been found to have a beneficial effect on TBE virus replication and clearly had given the virus a selective advantage in cell culture. On the other hand, this finding is consistent with earlier reports that other mutations in this same region have a deleterious effect on IRES function, and the approximately twofold reduction observed here is consistent with that observed previously with an A-to-C substitution at nucleotide 772 (Hoffman and Palmenberg, 1995).

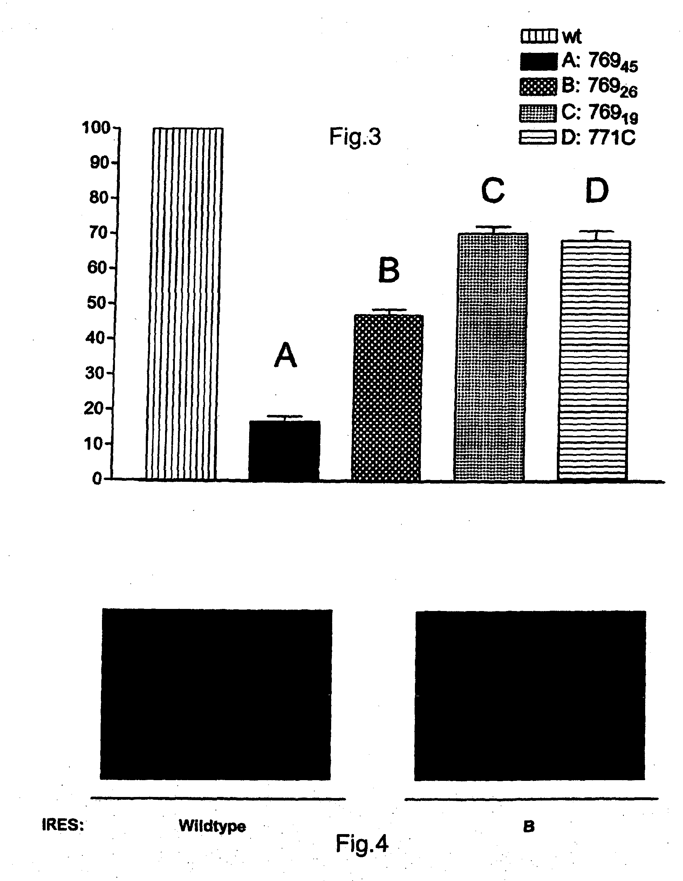

The translation efficiency of the group of mutants that had a U769A substitution followed by the insertion of multiple A residues was investigated. For this analysis, variants with 19, 26, and 45 A's in the oligoA loop was chosen to cover the range of insertion sizes that were found. These mutations were subcloned into the corresponding site of the pIRES2-eGFP reporter plasmid, and the effect of the mutations on eGFP expression was investigated by FACS analysis as described above for the A771C mutant. All three of these IRES were still functional. The IRES containing 19 A's in the oligo(A) loop was still about half as strong as the parent, and remarkably, even the IRES containing 45 A's still retained about 16% efficiency (see also Fig.3). Since the level of reporter gene expression in this experiment decreased as the number of inserted A's increased, it is clear that polyA insertions of different lengths are tolerated at this site but impair IRES function in proportion to the size of the insert.

### Influence of the A771C mutation on CAP- and IRES-dependent translation in the bicistronic TBEV construct:

The IRES in TBEV-bc, although normally a powerful translation initiation element in its natural context, works rather inefficiently in its position downstream from the TBEV CAP cistron. Expression of the TBEV prM and E proteins from the IRES was estimated to be 10-100 fold lower than when expressed as part of the normal polyprotein from the CAP-dependent initiation site (Orlinger et al., 2006). Therefore, the effect of the IRES mutations generated was analysed in this study on translation efficiency in the specific context of the artificial TBEV bicistronic genome.

To assess the levels of translation from each of the cistrons, the relative amounts of proteins NS1 (from the CAP cistron) and E (from the IRES cistron) were measured by FACS analysis using monoclonal antibodies. The A771C mutation, when present in the bicistronic TBEV genome, resulted in an overall increase in the levels of expression from both cistrons. Expression of NS1 from the CAP cistron increased about 2-fold relative to TBEV-bc, restoring it to wild-type levels. At the same time, expression of E protein from the IRES cistron increased about 3-9-fold relative to TBEV-bc. This result, at first glance, is paradoxical, because the previous experiment examining reporter gene expression showed that the mutation weakens the IRES itself. Nevertheless, it is consistent with the fact that viruses containing this mutation yielded higher titres and were selected in cell culture. It thus appears that whatever disadvantage is caused by the impaired IRES function is outweighed by an overall increased efficiency of the system that is conferred by the mutation.

A similar analysis of the U769/polyA mutants containing 19, 26, and 45 A's was done by cloning them back into the TBEV-bc construct and measuring NS1 and E expression as above. Relative to TBEV-bc, all of the U769A/polyA mutants showed a moderate increase in expression from the CAP-dependent cistron, restoring it to wild-type levels, as was also observed with the U771C mutation. This effect was the same regardless of the number of A's in the loop. In contrast, the number of A's in the IRES did have a strong effect on IRES-dependent expression of E protein, with expression levels strongly decreasing as the number of A's increased. Interestingly, these mutants showed different kinetics than the parental TBEV-bc construct, with the 19- and 26-A IRES insertion mutants producing higher levels of E protein than TBEV-bc for the first 2 to 3 days, after which they were surpassed by TBEV-bc.

The infectivity and virus particle yield of the 19-A mutant were approximately the same as with TBEV-bc. The 26- and 45-A mutants, however, were not as robust, and the 45-A construct was lost during further passage attempts. Interestingly, although the 26-A mutant initially gave lower titres than did TBEV-bc, its titre increased upon further passage, and the resulting virus population was again found to be a mixture containing adenine insertions of various lengths. It therefore appears that, while certain oligo(A) insertions are favoured, insertions that increase the length of the oligo(A) loop to significantly more than 19 A's are unstable and do not provide a long-term selective advantage.

### JK loop (=oligo (A) loop) mutations reduce translation efficiency of the IRES per se:

Translation efficiency of IRES elements with integrated oligo(A) mutations according to the present invention were analysed in a commercially available expression plasmid system (Clontech; pIRES2-eGFP). In this expression plasmid translation of a reporter gene (enhanced green fluorescence protein (eGFP)) is effected under the control of an EMCV IRES element. With standard cloning methods the IRES element of this vectors was exchanged with IRES elements carrying the mutations according to the present invention A, B, C, D according to Fig. 3.

These constructs and the vector with wild type IRES were electroporated into COS 1 cells and the level of expressed reporter gene was determined by FACS analysis.

This result shows a negative influence of the mutations according to the present invention on the translation efficiency of the IRES elements in the context of the expression plasmid.

### JK loop mutations improve the percentage of IF-positive cells after introduction of a bicistronic flavivirus vector:

The influence of the oligo(A) loop mutations according to the present invention on the introduction of flaviviral bicistronic RNA in host cells was analysed with immunofluorescence.

The same number of molecules of in vitro transcribed RNA of bicistronic flavivirus constructs with the oligo(A) mutations (B) and with wild type IRES was electroporated into BHK-21 cells. On day 2 after electroporation positive cells were detected by indirect immunofluorescence.

These results (Fig. 4) show a positive influence of the mutations according to the present invention with respect to efficiency of expression of gene products of bicistronic flavivirus vectors after introduction via electroporation into host cells.

### JK loop mutation raises the titre of a bicistronic infectious flavivirus:

A quantitation of viruses made by infectious bicistronic flaviviral constructs was performed in BHK-21 cells. In vitro transcribed RNA of flaviviral bicistronic constructs according to the present invention (D) and with wild type IRES was produced and the same amount of RNA molecules was electroproated into BHK-21 cells.

Six days after electroporation cell culture supernatants were cleared and diluted in half-logarithmic steps. With the concentrated supernatant and the various dilution steps fresh cells were infected. On day 3 after infection infected cells were detected by indirect immunofluorescence and the highest dilution step was identified which still contains infectious viruses.

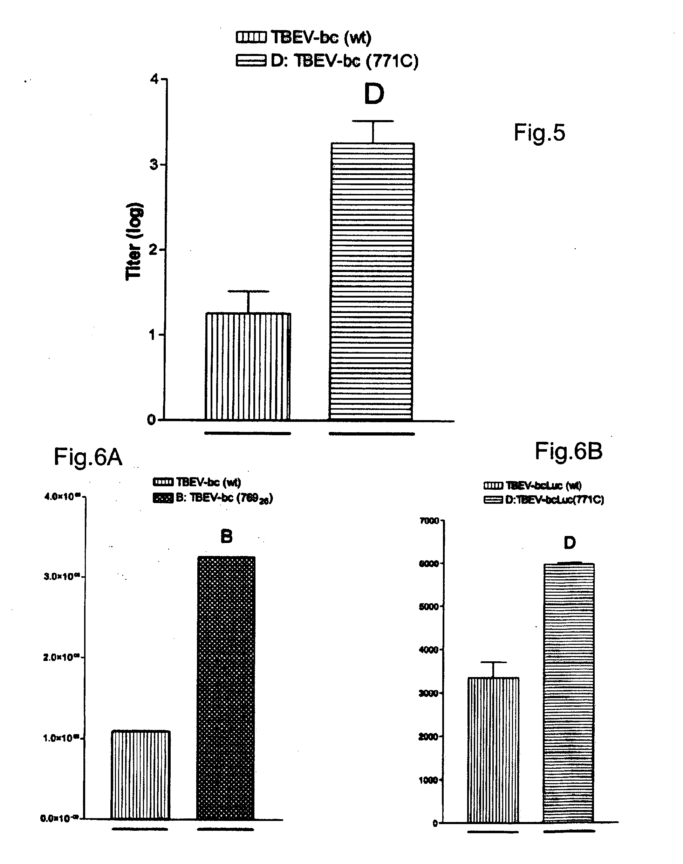

These results (Fig. 5) show a significantly positive effect of the mutated IRES sequences according to the present invention on the functionality of flaviviral bicistronic systems.

### JK loop mutations improve RNA replication of bicistronic flavivirus vectors:

The influence of the mutations according to the present invention on the replication capacity of bicistronic flaviviral constructs was determined by quantitative real time PCR (q real time PCR) and luciferase assay.

These experiments were conducted with bicistronic flaviviral constructs with the IRES mutations according to the present invention (B) and (D) wild type IRES.

The same amount of molecules of in vitro transcribed RNA of the constructs was electroporated into BHK-21 cells. The amount of RNA was determined after 18h by q real time PCR (Fig. 6A). In a further set-up the amount of the reporter gene luciferase was determined in an analogous manner 14h after electroporation (to correlate this amount of luciferase with the number of its template RNA which allows conclusions on the replication of the construct; Fig. 6B).

This result (Fig.6) shows the positive effect of the mutations according to the present invention on the replication efficiency of bicistronic flaviviral systems.

### JK loop mutations increase the export of infectious bicistronic flavivirus RNA:

The efficiency of packaging the bicistronic flaviviral constructs into viral particles was analysed with q real time PCR. Constructs with the IRES mutations according to the present invention (D) and wild type IRES RNA were in vitro transcribed and electroporated into BHK-21 cells. Six days after electroporation the amount of viral RNA exported from the cells was determined by quantitative real time PCR.

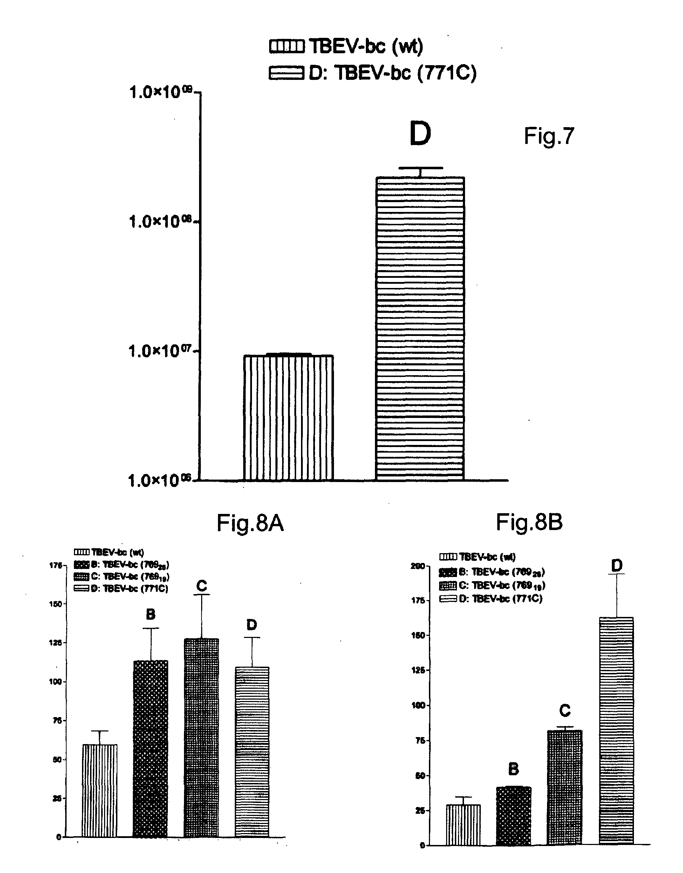

The results (Fig.7) show a significant positive influence of the mutations according to the present invention on the export of bicistronic flaviviral RNA from cells and indirectly proves the improvement in RNA replication and translation by the mutated IRES sequences according to the present invention.

### JK loop mutations enhance expression rate of both cistrons in the context of a bicistronic flavivirus vector:

An analysis of protein expression of constructs comprising the sequence amendments according to the present invention (B, C, D) in BHK-21 cells was performed by FACS analysis.

The expression level of the IRES cistron (Fig.8B) and the CAP cistron (Fig. 8A) was analysed. Representative proteins of both reading frames were marked with immunochemical methods and their amount was determined. As a control, an identical construct without IRES mutations was analysed. The amount of expressed proteins was compared. All constructs comprising the mutations according to the present invention showed a strongly enhanced expression of CAP and IRES translated gene products compared to vectors encoding wild_type IRES.

This result confirms a significant improvement of protein expression of both cistrons of bicistronic flaviviral vectors according to the present invention.

### Discussion

The ability to engineer infectious flavivirus clones to accommodate expression from two separate cistrons has a number of potentially useful applications, including their use as vehicles for the expression of foreign genes as well as tools for investigating the mechanisms of virus replication and assembly (Gehrke et al., 2005). In addition, the ability to produce infectious, self-propagating bicistronic flaviviruses has already been helpful in elucidating the details of the flavivirus replication and assembly processes (Orlinger et al., 2006). Here, it was demonstrated that the overall efficiency of replication of the artificial bicistronic TBE virus was improved by repeated passage in cell culture and also that a similar mutation could be selected in baby mouse brain.

Flaviviruses, like most other RNA viruses, have an error-prone RNA-dependent RNA polymerase that is capable of generating frequent random mutations throughout the genome. This results in quasispecies in which there is usually a diverse pool of mutants from which a particular variant can be selected under the appropriate conditions. It is therefore interesting that the only adaptive mutations that have been observed so far were either ones in the E protein that are frequently selected in cell culture to improve cell surface binding or in a very limited portion of the IRES. This suggests that these two factors were the main ones limiting the growth and proliferation of our bicistronic virus. Earlier, it was shown that adaptation of TBE virus to cell culture often involves an early selection of individual amino acid changes that increase the net positive charge of the E protein by one or two and produces a local patch of positive charge which is apparently responsible for increased affinity for heparan sulfate. Once one of these mutations has been selected, however, further mutations of this type are not usually observed in the same virus, suggesting that increasing the positive charge even more does not provide any further advantage.

In contrast, it is striking that the mutations that were selected with TBEV-bc were within the foreign IRES element. Since all of these mutations decreased the functional efficiency of the IRES, it is reasonable to assume that they were selected because they were able to compensate for a deleterious effect of having the IRES and/or the prM and E genes inserted into that particular position in the 3'-NCR. Because it is not yet clear which factors are limiting in the viral replicative cycle, it is not known whether the resulting lowered rate of translation of prM and E was actually harmful to the virus in this case, but it can be presumed that another factor, such as RNA replication efficiency, was probably more important for the selection process.

The functional organization of IRES elements has been extensively studied, and it is clear that the J-K stem-loop structure of the EMCV IRES is an important functional element and that the oligo(A) loop is directly involved in the association of the IRES with the cellular translation machinery. Using primer extension analysis as well as chemical and enzymatic protection experiments, it has been shown that this region serves as a binding site for eIF4G, an important translation factor. Changes in this loop have been shown to cause the IRES to become dependent on pyrimidine-tract-binding protein (PTBP) and also to strongly impair translation from the IRES (Hoffman and Palmenberg, 1995), consistent with the observations according to the present invention. Since the mutations that were selected in the bicistronic TBEV genome were of the type that would be expected to reduce the affinity of translation initiation factors for this region of the TBEV-bc genome, it can be concluded that it is these factors, or indirectly, the ribosome complexes that are recruited by these factors, that interfere with virus replication when bound tightly to the IRES. This, for example, could lower the amount of free template RNA that is available to the flavivirus RNA replication machinery. Furthermore, it has been observed with other positive-strand RNA viruses that when ribosomes, moving in the 5'-to-3' direction during translation, collide with the viral replication machinery moving in the opposite direction during minus-strand synthesis, translation continues but RNA replication stops. This implies that RNA replication can only occur when the RNA template is not monopolized by the translation machinery. A mutation that increases the initial rate of RNA replication at the expense of protein translation might therefore be particularly advantageous during the early stages of infection, especially if RNA template availability has become rate-limiting for the entire process.

It is also possible that competition for ribosomes between the CAP and IRES elements causes an imbalance at the translation level that results in suboptimal ratios of structural and non-structural proteins synthesized from different cistrons. Although this is a general problem of all bicistronic expression systems with interacting components, replicating viral vector systems would be expected to be especially sensitive in this respect. Currently, although it has been established that each virion has 180 copies each of E and M, little is known about the stoichiometry of the replication complexes or the nucleocapsid, or the relative amounts of the different structural and non-structural proteins that are required during the various stages of the replication cycle. The ability to make functional virus particles whose components are not derived from the same polyprotein might provide appropriate tools to address these questions.

The acquisition of variable numbers of additional adenine insertions in the oligo A loop of the IRES appears to be another way in which the bicistronic TBE virus can make further compensatory adjustments in the level of function of the foreign IRES, and it was observed that a mutant with a total of 19 A's in this loop was not significantly impaired in its ability to replicate and produce infectious particles (despite the fact that the rate of translation from the IRES was clearly lower), whereas mutants with even more A's (26 and 45) were less robust. If these larger insertions were actually selected in the present experiments, it is not completely clear what advantage they provided, especially since the isolated 26-A variant continued to mutate. However, the somewhat different kinetics of envelope protein expression that these mutants display when compared to TBEV-bc might provide a clue. In these experiments, the amount of E protein in the cells was higher for the first two to three days after transfection with the poly A mutants than with the parental bicistronic virus, and this might have provided an opportunity for the these mutants to gain an early, although temporary, advantage.

In practical terms, the present experiments have provided mutations that improve the efficiency of bicistronic flavivirus replication. Achieving the correct balance between the two translation units as well as the balance between translation and replication are key considerations in the development of artificial bicistronic viral expression systems.

## Claims

1. : Method for expressing proteins comprising
- providing a bicistronic flavivirus expression construct comprising a 5'-terminal CAP structure, a 5'-non-coding sequence, a sequence encoding non-structural proteins, an internal ribosomal entry site (IRES) comprising an oligo(A) loop, a sequence encoding the protein to be expressed and a 3'-non-coding sequence in a suitable host cell and
- incubating this host cell with the expression construct to allow the protein to be expressed,
**characterised in that** the IRES carries a mutation in the oligo(A) loop.

2. : Method according to claim 1, **characterised in that** the oligo(A) loop is defined as consisting of base 769 to 776 of IRES of Encephalomyocarditis virus (EMCV) in the J-K stem loop.

3. : Method according to claim 1 or 2, **characterised in that** the protein to be expressed is a non-flavivirus protein.

4. : Method according to any one of claims 1 to 3, **characterised in that** the construct comprises one or more structural protein(s) of the flavivirus.

5. : Method according to any one of claims 1 to 4, **characterised in that** the protein to be expressed is translated in a CAP dependent manner or in an IRES dependent manner.

6. : Method according to any one of claims 1 to 5, **characterised in that** the mutation in the oligo(A) loop is selected from the group consisting of any base substitution of U769, A770, A771, A 772, an insertion of 1 to 50 nucleic acid residues at any base from U769 to A776, or combinations thereof.

7. : Method according to any one of claims 1 to 6, **characterised in that** the mutation in the oligo(A) loop is selected from the group consisting of U769A, A770U, A770C, A771U, A771C, A 772U, A772C, an insertion of 1 to 37 A residues at any base from U769 to A776, or combinations thereof.

8. : Method according to any one of claims 1 to 7, **characterised in that** the mutation in the oligo(A) loop is selected from the group consisting of U769A, A771U, A771C, or an insertion of 1 to 10 A residues at any base from U769 to A776.

9. : Method according to any one of claims 1 to 8, **characterised in that** the construct further comprises sequences encoding flavivirus surface proteins prM and E.

10. : Method according to any one of claims 1 to 9, **characterised in that** the host cell is selected from the group consisting of baby hamster kidney cells, Vero cells, Chinese hamster ovary cells, HeLa cells and Hep cells.

11. : Method according to any one of claims 1 to 10, **characterised in that** the protein to be expressed is selected from the group consisting of exogenous polypeptides from pathogenic viruses, bacteria, yeast, fungi and protozoa, tumor associated antigens, "self" antigens or proteins with therapeutic activity, especially cytokines, hormones, bioactive peptides, soluble forms of membrane proteins, enzymes, regulatory proteins, structural proteins, clotting factors, chemokines, erythropoietin or t-PA.

12. : Method according to any one of claims 1 to 11, **characterised in that** the IRES is selected from the group consisting of IRES form EMCV, Theilers Murine encephalomyelitis virus, Equine rhinits virus A or B, Mengo virus and Foot-and-mouth disease virus (FMDV).

13. : Method for improving the yield of expressed protein in a bicistronic flavivirus expression system comprising
- providing a bicistronic flavivirus expression construct comprising a 5'-terminal CAP structure, a 5'-non-coding sequence, a sequence encoding non-structural proteins, an internal ribosomal entry site (IRES) comprising an oligo(A) loop, 3' non-coding sequences and a sequence encoding the protein to be expressed in a suitable host cell and
- incubating this host cell with the expression construct to allow the protein to be expressed,
**characterised in that** an IRES with a mutation in the oligo(A) loop is used.

14. : Use of an IRES with a mutation in the oligo(A) loop for improving the expression yield of a protein expressed in a flavivirus bicistronic expression system.

15. : IRES with a mutation selected from the group consisting of U769A, A771U, A771C, or an insertion of 1 to 10 A residues at any base from U769 to A776.

16. : Replication competent bicistronic flavivirus expression construct comprising a mutation in the oligo(A) loop of the IRES.

17. : Replication competent bicistronic flavivirus expression vector comprising a mutation in the oligo(A) loop of the IRES and a coding sequence for an non-flavivirus protein to be expressed.

18. : Infectious recombinant bicistronic flavivirus comprising an IRES, **characterised in that** the IRES comprises a mutation in the oligo(A) loop.

19. : Flavivirus according to claim 18, **characterised in that** it is selected from yellow fever virus (YFV), Dengue virus serotypes 1 to 4, Japanese encephalitis virus, West Nile virus, Kunjin virus or Tick-borne encephalitis virus.

20. : Host cell comprising a flavivirus according to claims 18 or 19.

21. : Nucleic acid molecule encoding a replication competent bicistronic flavivirus expression construct comprising a mutation in the oligo(A) loop of the IRES.

22. : Use of a nucleic acid according to claim 21 for the manufacture of a pharmaceutical composition, especially an immunotherapeutic composition or a vaccine.
